(19) Europäisches Patentamt
European Patent Office
Office européen des brevets

(11) Veröffentlichungsnummer : **0 177 740**
**B1**

# (12) EUROPÄISCHE PATENTSCHRIFT

(45) Veröffentlichungstag der Patentschrift : **28.09.88**

(51) Int. Cl.⁴ : **C 07 D275/06**, A 01 N 47/38

(21) Anmeldenummer : **85110995.9**

(22) Anmeldetag : **31.08.85**

## (54) Acylierte Saccharin-Derivate.

(30) Priorität : **12.09.84 DE 3433391**

(43) Veröffentlichungstag der Anmeldung : **16.04.86 Patentblatt 86/16**

(45) Bekanntmachung des Hinweises auf die Patenterteilung : **28.09.88 Patentblatt 88/39**

(84) Benannte Vertragsstaaten :
**AT BE CH DE FR GB IT LI NL SE**

(56) Entgegenhaltungen :
**FR-A- 2 507 599**
**GB-A- 1 278 111**
**US-A- 3 264 314**
**Die Akte enthält technische Angaben, die nach dem Eingang der Anmeldung eingereicht wurden und die nicht in dieser Patentschrift enthalten sind.**

(73) Patentinhaber : **BAYER AG**
**Konzernverwaltung RP Patentabteilung**
**D-5090 Leverkusen 1 Bayerwerk (DE)**

(72) Erfinder : **Salzburg, Herbert, Dr.**
**Hahnenweg 3**
**D-5000 Köln 80 (DE)**
Erfinder : **Hajek, Manfred, Dr.**
**Hahnenweg 1**
**D-5000 Köln 80 (DE)**
Erfinder : **Hagemann, Hermann, Dr.**
**Kandinskystrasse 52**
**D-5090 Leverkusen 1 (DE)**
Erfinder : **Kühle, Engelbert, Dr.**
**von-Bodelschwingh-Strasse 42**
**D-5060 Bergisch-Gladbach 2 (DE)**
Erfinder : **Führer, Wolfgang, Dr.**
**Wehrstrasse 28**
**D-5202 Hennef 1 (DE)**
Erfinder : **Hänssler, Gerd, Dr.**
**Am Arenzberg 58a**
**D-5090 Leverkusen 3 (DE)**
Erfinder : **Brandes, Wilhelm, Dr.**
**Eichendorffstrasse 3**
**D-5653 Leichlingen 1 (DE)**
Erfinder : **Reinecke, Paul, Dr.**
**Steinstrasse 8**
**D-5090 Leverkusen 3 (DE)**



Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft neue acylierte Saccharin-Derivate, ein Verfahren zu ihrer Herstellung und ihre Verwendung in Schädlingsbekämpfungsmitteln.

Es ist bereits bekannt, daß Alkylaminocarbonyl-saccharin-Derivate, z. B. das N-[Methylaminocarbonyl]-saccharin, eine fungizide Wirksamkeit aufweisen (vgl. z. B. DE-OS-1 953 422). Weiterhin sind halogenierte Phenylaminocarbonyl-saccharin-Derivate, wie z. B. das N-[3,4-Dichlorphenylaminocarbonyl]-saccharin, bekannt und ihre bakterizide und fungizide Wirkung (vgl. US-PS-3 264 314).

Weiterhin sind 3-Alkenyloxy-isosaccharin-Derivate und ihre fungizide Wirksamkeit bekannt (vgl. JP-7 014 301).

Weiterhin sind substituierte Aminocarbonylsaccharide, wie z. B. N-Methylaminocarbonyl-saccharin, als Bakterizide im Pflanzenschutz bekannt (vgl. FR-A—2 507 599).

Es wurden neue acylierte Saccharin-Derivate der allgemeinen Formel (I)

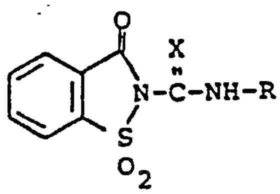

(I)

in welcher

X für Sauerstoff oder Schwefel steht,

R für die Gruppierung —CO—$R^1$ oder —$SO_2$—$OR^2$ steht, wobei

$R^1$ für geradkettiges oder verzweigtes Alkyl mit 1 bis 4 Kohlenstoffatomen, für geradkettiges oder verzweigtes Halogenalkyl oder Halogenalkoxy mit jeweils 1 bis 6, insbesondere 1 bis 4 Kohlenstoffatomen und 1 bis 5, insbesondere 1 bis 3 gleichen oder verschiedenen Halogenatomen, für geradkettiges oder verzweigtes Alkoxy oder Alkylthio mit jeweils 1 bis 10 Kohlenstoffatomen, für jeweils gegebenenfalls 1- bis 5-fach, gleich oder verschieden substituiertes Aryl, Aryloxy oder Arylthio mit jeweils 6 bis 10 Kohlenstoffatomen, wobei als Substituenten des Aryls genannt seien : Halogen, geradkettiges oder verzweigtes Alkoxy mit 1 bis 4 Kohlenstoffatomen, Alkoxycarbonyl mit 1 bis 4 Kohlenstoffatomen im Alkoxyteil, geradkettiges oder verzweigtes Alkyl mit 1 bis 4 Kohlenstoffatomen und N-Halogenalkyl-N-halogenalkylthioamino mit 1 bis 3 Kohlenstoffatomen und 1 bis 5 gleichen oder verschiedenen Halogenatomen je Halogenalkylrest, ferner für Cycloalkoxy mit 3 bis 6 Kohlenstoffatomen oder für die Gruppe —$NR^3R^4$ steht,

$R^2$ für geradkettiges oder verzweigtes Alkyl mit 1 bis 4 Kohlenstoffatomen oder Phenyl steht,

$R^3$ für geradkettiges oder verzweigtes Alkyl mit 1 bis 8 Kohlenstoffatomen steht und

$R^4$ für geradkettiges oder verzweigtes Alkyl mit 1 bis 4 Kohlenstoffatomen, Phenyl, Halogenalkylthio mit 1 bis 4 Kohlenstoffatomen und 1 bis 5 gleichen oder verschiedenen Halogenatomen, Alkoxycarbonyl mit 1 bis 4 Kohlenstoffatomen im Alkoxyteil oder Phenoxycarbonyl steht oder

$R^3$ und $R^4$ gemeinsam mit dem Stickstoffatomen, an dem sie stehen, einen Saccharinrest bilden, gefunden.

Weiterhin wurde gefunden, daß man die neuen acylierten Saccharin-Derivate der allgemeinen Formel (I)

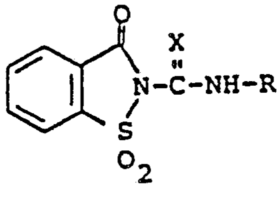

(I)

in welcher

X für Sauerstoff oder Schwefel steht,

R für die Gruppierung —CO—$R^1$ oder —$SO_2$—$OR^2$ steht, wobei

$R^1$ für geradkettiges oder verzweigtes Alkyl mit 1 bis 4 Kohlenstoffatomen, für geradkettiges oder verzweigtes Halogenalkyl oder Halogenalkoxy mit jeweils 1 bis 6, insbesondere 1 bis 4 Kohlenstoffatomen und 1 bis 5, insbesondere 1 bis 3 gleichen oder verschiedenen Halogenatomen, für geradkettiges oder verzweigtes Alkoxy oder Alkylthio mit jeweils 1 bis 10 Kohlenstoffatomen, für jeweils gegebenenfalls 1- bis 5-fach, gleich oder verschieden substituiertes Aryl, Aryloxy oder Arylthio mit jeweils 6 bis 10 Kohlenstoffatomen, wobei als Substituenten des Aryls genannt seien : Halogen, geradkettiges oder verzweigtes Alkoxy mit 1 bis 4 Kohlenstoffatomen, Alkoxycarbonyl mit 1 bis 4 Kohlenstoffatomen im Alkoxyteil, geradkettiges oder verzweigtes Alkyl mit 1 bis 4 Kohlenstoffatomen und N-Halogenalkyl-N-halogenalkylthioamino mit 1 bis 3 Kohlenstoffatomen und 1 bis 5 gleichen oder verschiedenen Halogenatomen je Halogenalkylrest, ferner für Cycloalkoxy mit 3 bis 6 Kohlenstoffatomen oder für die Gruppe —$NR^3R^4$ steht,

$R^2$ für geradkettiges oder verzweigtes Alkyl mit 1 bis 4 Kohlenstoffatomen oder Phenyl steht,

$R^3$ für geradkettiges oder verzweigtes Alkyl mit 1 bis 8 Kohlenstoffatomen steht und

$R^4$ für geradkettiges oder verzweigtes Alkyl mit 1 bis 4 Kohlenstoffatomen, Phenyl, Halogenalkylthio mit 1 bis 4 Kohlenstoffatomen und 1 bis 5 gleichen oder verschiedenen Halogenatomen, Alkoxycarbonyl mit 1 bis 4 Kohlenstoffatomen im Alkoxyteil oder Phenoxycarbonyl steht oder

$R^3$ und $R^4$ gemeinsam mit dem Stickstoffatom, an dem sie stehen, einen Saccharinrest bilden, erhält, wenn man Saccharin der Formel (II)

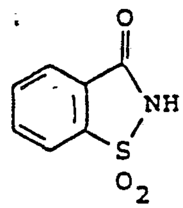

(II)

mit Iso- bzw. Thioisocyanat-Derivaten der Formel (III)

$$XCN—R \qquad (III)$$

in welcher

X und R die oben angegebene Bedeutung haben, gegebenenfalls in Gegenwart eines Lösungs- oder Verdünnungsmittels und gegebenenfalls in Gegenwart eines Katalysators umsetzt.

Schließlich wurde gefunden, daß die neuen acylierten Saccharin-Derivate eine gute Wirkung gegen Schädlinge besitzen, vor allem gegen Pilze und Bakterien.

Überraschenderweise zeigen die erfindungsgemäßen acylierten Saccharin-Derivate der Formel (I) eine höhere biologische Wirksamkeit als die aus dem Stand der Technik vorbekannten Verbindungen der gleichen Wirkungsrichtung. Die erfindungsgemäßen Verbindungen stellen somit eine Bereicherung der Technik dar.

Die erfindungsgemäßen acylierten Saccharin-Derivate sind durch die Formel (I) allgemein definiert. Bevorzugt sind Verbindungen der Formel (I), bei welchen

X für Sauerstoff oder Schwefel steht,

R für die Gruppierung —CO—$R^1$ oder —$SO_2$—$OR^2$ steht, wobei

$R^1$ für Methyl, Ethyl, für geradkettiges oder verzweigtes Halogenalkyl oder Halogenalkoxy mit jeweils 1 bis 3 Kohlenstoffatomen und 1 bis 3 gleichen oder verschiedenen Fluor- und Chloratomen, für geradkettiges oder verzweigtes Alkoxy mit 1 bis 10 Kohlenstoffatomen, für geradkettiges oder verzweigtes Alkylthio mit 1 bis 4 Kohlenstoffatomen, für jeweils gegebenenfalls ein- bis dreifach, gleich oder verschieden durch Chlor, Fluor, Methoxy, Ethoxy, Methyl, Ethyl, Methoxycarbonyl, Ethoxycarbonyl und N-Trifluormethyl-N-fluordichlormethylthioamino substituiertes Phenyl, Phenoxy oder Phenylthio, für Cyclohexoxy oder für die Gruppe —$NR^3R^4$ steht,

$R^2$ für Methyl, Ethyl oder Phenyl steht,

$R^3$ für geradkettiges oder verzweigtes Alkyl mit 1 bis 5 Kohlenstoffatomen steht,

$R^4$ für Methyl, Ethyl, Phenyl, Phenoxycarbonyl, Methoxycarbonyl, Ethoxycarbonyl oder Halogenalkylthio mit 1 bis 3 Kohlenstoffatomen und 1 bis 3 gleichen oder verschiedenen Fluor- und Chloratomen steht oder

$R^3$ und $R^4$ mit dem Stickstoffatom, an dem sie stehen, einen Saccharinrest bilden.

Ganz besonders bevorzugt sind die Verbindungen der Formel (I), bei welchen

X für Sauerstoff steht,

R für die Gruppierung —CO—$R^1$ steht, wobei

$R^1$ für Methoxy, Ethoxy, n-Propoxy, iso-Propoxy, n-Butoxy, iso-Butoxy, tert.-Buitoxy, Pentoxy, Hexoxy, 2-n-Butyl-butoxy, Chlormethoxy, 2,2,2-Trifluorethoxy, 1-Chlormethyl-2-chlorethoxy, Methylthio, Ethylthio, n-Propylthio, iso-Propylthio, Phenoxy, Phenylthio, Cyclohexoxy, 2-Methoxy-phenoxy, 4-Methoxy-phenoxy, 2-Chlor-phenoxy, 4-Chlorphenoxy, 2,4-Dichlorphenoxy, 2-Methoxycarbonylphenoxy, 2-Chlorphenylthio, 4-Chlorphenylthio, N-Methyl-N-phenylamino, N-Ethyl-N-phenylamino, N-n-Propyl-N-phenyl-amino, N-n-Butyl-N-phenylamino, N-n-Butyl-N-trichlormethylthio-amino, N-Methyl-N-phenoxycarbonyl-amino, N-Ethyl-N-phenoxy-carbonyl-amino, N-n-Propyl-N-phenoxy-carbonyl-amino, N-Methyl-N-methoxy-carbonyl-amino, N-Ethyl-N-ethoxy-carbonyl-amino, N-n-Butyl-N-phenoxy-carbonyl-amino, N-iso-Butyl-N-phenoxy-carbonyl-amino, N-iso-Pentyl-N-phenoxy-carbonyl-amino, 2-[N-Trifluormethyl-N-dichlor-fluormethylthio-amino]-phenyl, 3-[N-Trifluormethyl-N-dichlorfluormethylthio-amino]-phenyl, 4-[N-Trifluormethyl-N-dichlorfluormethylthio-amino]-phenyl oder 1,1-Dioxid-3-keto-2H,3H-1,2-benzisothiazolyl steht oder

X für Sauerstoff steht,
R für die Gruppierung $—SO_2—OR^2$ steht, wobei
$R^2$ für Phenyl, Methyl oder Ethyl steht.

Verwendet man beispielsweise Saccharin und Methoxycarbonylisocyanat bzw. Dimethylaminocarbonylisocyanat als Ausgangsmaterialien, so lassen sich die Reaktionsabläufe durch die folgenden Formelschemata wiedergeben :

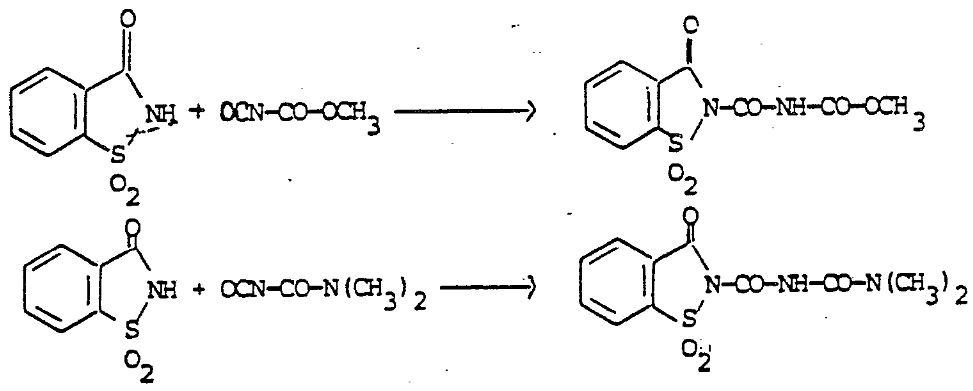

Das zur Durchführung des erfindungsgemäßen Verfahrens als Ausgangsstoff benötigte Saccharin der Formel (II) ist bekannt une käuflich auf in technischem Maßstab zu erwerben.

Die weiterhin als Ausgangsstoffe benötigten Iso- bzw. Thioisocyanat-Derivate der Formel (III) sind ebenfalls bekannt und nach literaturbekannten Verfahren herstellbar. Allgemeine Vorschriften sind in Standardwerken wie Houben-Weyl « Methoden der Organischen Chemie » E 4, 1983, Thieme-Verlag Stuttgart beschrieben, Verbindungen, in denen R für z. B. Alkylcarbonyl steht, können hergestellt werden wie beschrieben in « J. Org. Chem. » 27, 3742ff (1962) und 28, 1805ff (1962), außerdem « Angewandte Chemie » 89, 789 (1977). Verbindungen in denen R für z. B. Alkoxycarbonyl steht, können nach den Beschreibungen in « Berichte der Deutschen Chem. Ges. » 39, 688ff (1906) hergestellt werden.

Als Verdünnungsmittel kommen für das Verfahren alle inerten organischen Lösungsmittel in Frage. Hierzu gehören vorzugsweise aliphatische oder aromatische, gegebenenfalls halogenierte Kohlenwasserstoffe, wie beispielsweise Benzin, Benzol, Toluol, Xylol, Petrolether, Pentan, Hexan, Cyclohexan, Dichlormethan, Chloroform, Tetrachlorkohlenstoff oder Chlorbenzol ; Ether, wie Diethylether, Diisopropylether, Ethylenglycoldimethylether, Dioxan oder Tetrahydrofuran ; Ketone, wie Aceton oder Butanon ; Nitrile, wie Acetonitril oder Propionitril, Ester von Carbonsäuren, wie Essigsäureethylester, Essigsäurebutylester oder Propionsäureethylester.

Das erfindungsgemäße Verfahren kann auch in Abwesenheit eines Verdünnungs- oder Lösungsmittels durchgeführt werden. In diesem Fall setzt man vorzugsweise die Acylierungskomponente in einem Überschuß von 0,1 bis 5 Äquivalenten ein. Nach Reaktionsbeendigung wird das überschüssige Reagenz im Vakuum abdestilliert.

Die Reaktionstemperaturen können bei der Durchführung des erfindungsgemäßen Verfahrens in einem größeren Bereich variiert werden. Im allgemeinen arbeitet man zwischen 0 °C und 100 °C, vorzugsweise zwischen 25 °C und 60 °C.

Das erfindungsgemäße Verfahren wird vorzugsweise bei Normaldruck durchgeführt.

Als Katalysatoren werden die für Isocyanat-Additionen üblichen Katalysatoren, wie z. B. Dibutylzinndilaurat, Triethylamin und Triethylendiamin (DABCO), eingesetzt.

Eine variante der Reaktions besteht darin, daß anstelle der Alkoxycarbonylisocyanate ein Alkoxycarbonylaminocarbonylhalogenid vorzugsweise -chlorid, eingesetzt werden kann. Zweckmäßigerweise setzt man auch hier ungefähr ein Äquivalent einer Hilfsbase wie Triethylamin oder Pyridin, Natriumhydrogencarbonat oder Natriumcarbonat zu.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Schädlingsbekämpfungsmittel geeignet.

So werden z. B. fungizide Mittel im Pflanzenschutz eingesetzt zur Bekämpfung von Plasmodiophoromycetes, Oomycetes, Chytridiomycetes, Zygomycetes, Ascomycetes, Basidiomycetes, Deuteromycetes.

Bakterizide Mittel werden z. B. im Pflanzenschutz zur Bekämpfung von Pseudomonadaceae, Rhizobiaceae, Enterobacteriaceae, Corynebacteriaceae und Streptomycetaceae eingesetzt.

Beispielhaft, aber nicht begrenzend, seien einige Erreger von pilzlichen und bakteriellen Erkrankungen, die unter die oben aufgezählten Oberbegriffe fallen, genannt :

Botrytis-Arten, wie beispielsweise Botrytis cinerea ;
Plasmopara-Arten, wie beispielsweise Plasmopara viticola ;
Uromyces-Arten, wie beispielsweise Uromyces appendiculatus ;
Sphaerotheca-Arten, wie beispielsweise Sphaerotheca fuliginea ;

Venturia-Arten, wie beispielsweise Venturia inaequalis ;
Podosphaera-Arten, wie beispielsweise Podosphaera leucotricha ;
Phytophthora-Arten, wie beispielsweise Phytophthora infestans ;
Erysiphe-Arten, wie beispielsweise Erysiphe graminis ;
Puccinia-Arten, wie beispielsweise Puccinia recondita ;
Fusarium-Arten, wie beispielsweise Fusarium culmorum ;
Ustilago-Arten, wie beispielsweise Ustilago nuda oder Ustilago avenae ;
Septoria-Arten, wie beispielsweise Septoria nodorum ;
Tilletia-Arten, wie beispielsweise Tilletia caries ;
Xanthomonas-Arten, wie beispielsweise Xanthomonas oryzae ;
Pseudomonas-Arten, wie beispielsweise Pseudomonas lachrymans ;
Pyricularia-Arten, wie beispielsweise Pyricularia oryzae ;
Pellicularia-Arten, wie beispielsweise Pellicularia sasakii ;
Erwinia-Arten, wie beispielsweise Erwinia amylovova ;
Pyrenophora-Arten, wie beispielsweise Pyrenophora teres ; (Konidienform : Drechslera, Syn : Helminthosporium) ;
Leptosphaeria-Arten, wie beispielsweise Leptosphaeria nodorum ;
Cochliobolus-Arten, wie beispielsweise Cochliobolus sativus (Konidienform : Drechslera, Syn : Helminthosporium) und
Cercospora-Arten, wie beispielsweise Cercospora canescens.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Schädlingsbekämpfungsmittel können die erfindungsgemäßen Wirkstoffe mit besonders guten Erfolg zur Bekämpfung von Obst- und Gemüsekrankheiten, wie beispielsweise gegen den Erreger der Braunfäule (Phytophthora infestans) an Tomaten, außerdem zur Bekämpfung von Getreidekrankheiten, zum Beispiel verursacht durch Pyrenophora teres, Septoria nodorum, Fusarium culmorum und Drechslera graminea, weiterhin zur Bekämpfung von Reiskrankheiten, wie beispielsweise den Erreger der Blattfleckenkrankheit (Pyricularia oryzae) eingesetzt werden. Die Verbindungen weisen auch eine bakterizide wirkung auf.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage : Aromaten, wie Xylol, Toluol oder Alkylnaphthaline, chlorierte Aromaten oder chlorierte aliphatische Kohlenxwasserstoffe, wie Chlorbenzole, Chlorethylene oder Methylenchlorid, aliphatische Kohlenwasserstoffe, wie Cyclohexan oder Paraffine, z. B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgase, wie Halogenkohlenwasserstoffe sowie Butan, Propan, Stickstoff und Kohlendioxid. Als feste Trägerstoffe kommen in Frage : z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage : z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und/oder schaumerzeugende Mittel kommen in Frage : z. B. nichtionogene und anionische Emulgatoren, wie Polyoxyethylen-Fettsäureester, Polyoxyethylen-Fettalkoholether, z. B. Alkylarylpolyglycol-ether, Alkylsulfonate, Alkylsulfate, Arylsulfonate sowie Eiweißhydrolysate. Als Dispergiermittel kommen in Frage : z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z. B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff,

vorzugsweise zwischen 0,5 und 90 %.

Die erfindungsgemäßen Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und Wachstumsregulatoren.

Die Wirkstoffe können als solche, in Form ihrer Formulierungen oder den daraus bereiteten Anwendungsformen, wie gebrauchsfertige Lösungen, emulgierbare Konzentrate, Emulsionen, Schaüme, Suspensionen, Spritzpulver, Pasten, lösliche Pulver, Stäubemittel und Granulate, angewendet werden. Die Anwendung geschieht in üblicher Weise, z. B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.

## Herstellungsbeispiele

### Beispiel 1

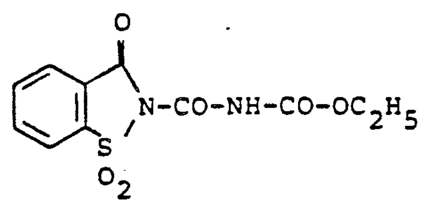

17,3 g (0,095 Mol) Saccharin werden in 250 ml Aceton mit 0,2 ml Triethylamin und anschließend unter Zutropfen mit 11,5 g (0,1 Mol) Ethoxycarbonylisocyanat versetzt. Nach 1 h Rühren bei 45 °C ist die Umsetzung beendet. Einengen in Wakuum liefert 28,2 g (99 % der Theorie) an N-(Ethoxycarbonylaminocarbonyl)-saccharin vom Schmp. 148 °C.

### Beispiel 2

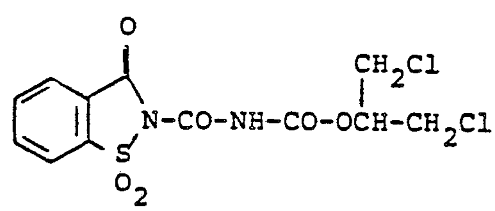

Analog Beispiel 1 erhält man aus 17,3 g Saccharin und 19,7 g (0,1 Mol) 1,3-Dichlor-isopropyl-2-oxycarbonyl-isocyanat in 200 ml Methylenchlorid 36,0 g (99 % der Theorie) an N-(1,3-Dichlorisopropyl-2-oxycarbonylaminocarbonyl)-saccharin mit einem Schmelzpunkt von 208 °C.

### Beispiel 3

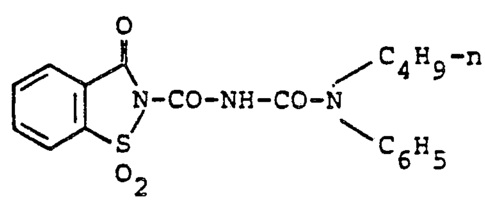

9,2 g Saccharin (0,05 Mol) werden in 100 ml Dioxan mit 0,1 g Triethylendiamin (DABCO) gelöst und anschließend 10,9 g (0,05 Mol) Butylphenylaminocarbonylisocyanat zugegeben. Das Gemisch wird unter Feuchtigkeitsausschluß 6 h Rückfluß gekocht, anschließend eingeengt und der Rückstand dreimal mit Diisopropylether verrührt. Man erhält 6 g (30 % der Theorie) an n-Butyl-phenylaminocarbonylaminocarbonylsaccharin mit dem Schmelzpunkt von 210 °C.

In entsprechender Weise gemäß den allgemeinen Herstellungsangaben werden die folgenden Verbindungen der Formel (I) hergestellt :

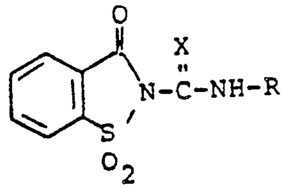

(I)

| Beispiel-Nr. | X | R | Physikalische Konstanten [Schmp: °C] |
|---|---|---|---|
| 4 | O | $-CO-OCH_2-CH(CH_3)_2$ | 146 |
| 5 | O | $-CO-OCH_2-CF_3$ | 181 |
| 6 | O | $-CO-SC_3H_7-n$ | 179 |
| 7 | O | $-CO-OCH_2-CH-CH_2-CH_3$<br>$(CH_2)_3-CH_3$ | Öl |
| 8 | O | 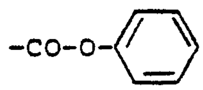 | 112 |
| 9 | S | $-CO-OC_2H_5$ | 243 |
| 10 | O | 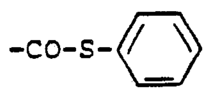 | 180 |
| 11 | O | $-CO-OCH_2Cl$ | 167 |
| 12 | O | $-CO-OC_6H_{11}$ | 118 |
| 13 | O | 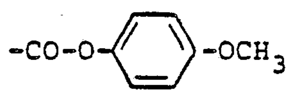 | Öl |
| 14 | O | $-CO-OC_4H_9-n$ | 224 |
| 15 | O | 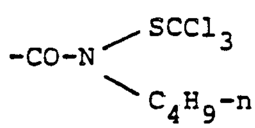 | Öl |
| 16 | O | 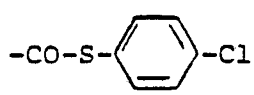 | Öl |
| 17 | O | 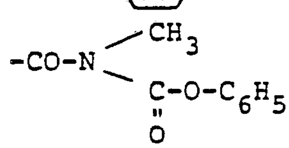 | 190 |
| 18 | O | 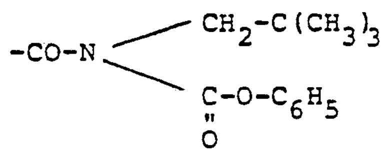 | Öl |

(Fortsetzung)

| Beispiel-Nr. | X | R | Physikalische Konstanten [Schmp: °C] |
|---|---|---|---|
| 19 | O | 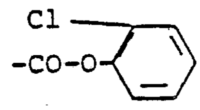 | 194 |
| 20 | O | 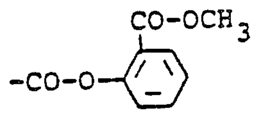 | 175 |
| 21 | O | 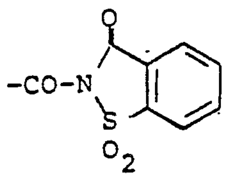 | 265 |
| 22 | S | $-CO-OC_6H_5$ | 210 |
| 23 | O | $-SO_2-O-C_6H_5$ | 161 |
| 24 | O | 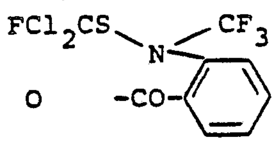 | 98 |
| 25 | | 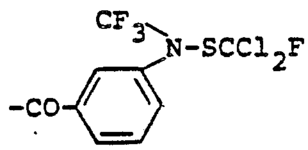 | 198 |
| 26 | O | 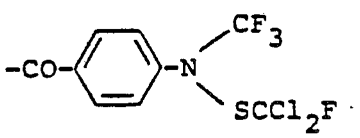 | 81 |

## Anwendungsbeispiele

In den folgenden Anwendungsbeispielen werden die nachfolgend aufgeführten Verbindungen als Vergleichssubstanzen eingesetzt :

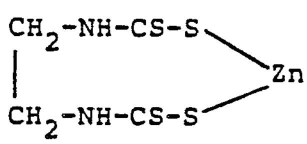

(A)

Zinkethylen-1,2-bis-dithiocarbamat

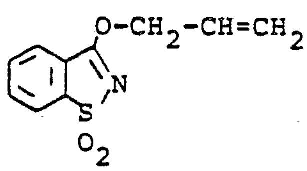

(B)

3-Allyloxy-1,2-benzisothiazol-1,1-dioxid

$$(CH_3)_2N-SO_2-\underset{\displaystyle C_6H_5}{N}-SCCl_2F$$

N,N-Dimethyl-N'-phenyl-N'-dichlorfluor-methylthio-sulfamid.

Beispiel A

Pyricularia-Test (Reis)/protektiv
Lösungsmittel : 12,5 Gewichtsteile Aceton
Emulgator : 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25 °C aufgestellt.

4 Tage nach der Inokulation erfolgt die Auswertung des Krankheitsbefalls.

Eine deutliche Überlegenheit in der Wirksamkeit gegenüber dem Stand der Technik zeigen in diesem Test z. B. die Verbindungen gemäß den Herstellungsbeispielen : 2, 14, 8, 5, 23, 10, 1, 13, 16, 15 und 24.

Beispiel B

Pyricularia-Test (Reis)/systemisch
Lösungsmittel : 12,5 Gewichtsteile Aceton
Emulgator : 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf systemische Eigenschaften werden 40 ml der Wirkstoffzubereitung auf Einheitserde gegossen, in der junge Reispflanzen angezogen wurden. 7 Tage nach der Behandlung werden die Pflanzen mit einer wäßrigen Sporensuspension von Pyricularia oryzae inokuliert. Danach verbleiben die Pflanzen in einem Gewächshaus bei einer Temperatur von 25 °C und einer rel. Luftfeuchtigkeit von 100 % bis zur Auswertung.

4 Tage nach der Inokulation erfolgt die Auswertung des Krankheitsbefalls.

Eine deutliche Überlegenheit in der Wirksamkeit gegenüber dem Stand der Technik zeigen in diesem Test z. B. die Verbindungen gemläß den Herstellungsbeispielen : 2, 14, 8, 5, 4, 23, 10, 11, 13, 16, 15 und 26.

Beispiel C

Phytophthora-Test (Tomate)/protektiv
Lösungsmittel : 4,7 Gewichtsteile Aceton
Emulgator : 0,3 Gewichtsteile Alkyl-aryl-polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension von Phytophthora infestans inokuliert.

Die Pflanzen werden in einer Inkubationskabine mit 100 % relativer Luftfeuchtigkeit und ca. 20 °C aufgestellt.

3 Tage nach der Inokulation erfolgt die Auswertung.

Eine deutliche Überlegenheit in der Wirksamkeit gegenüber dem Stand der Technik zeigen in diesem Test z. B. die Verbindungen gemäß den Herstellungsbeispielen : 25 und 24.

Beispiel D

Pyrenophora teres-Test (Gerste)/protektiv
Lösungsmittel : 100 Gewichtsteile Dimethylformamid
Emulgator : 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Pyrenophora teres besprüht. Die Pflanzen verbleiben 48 Stunden bei 20 °C und 100 % rel. Luftfeuchtigkeit in einer Inkubationskabine.

Die Pflanzen werden in einem Gewächshaus bei einer Temperatur von ca. 20 °C und einer relativen Luftfeuchtigkeit von ca. 80 % aufgestellt.

7 Tage nach der Inokulation erfolgt die Auswertung.

Eine deutliche Überlegenheit in der Wirksamkeit gegenüber dem Stand der Technik zeigt bei diesem Test z. B. die Verbindung gemäß dem Herstellungsbeispiel : 24.

**Patentansprüche**

1. Acylierte Saccharin-Derivate der allgemeinen Formel (I)

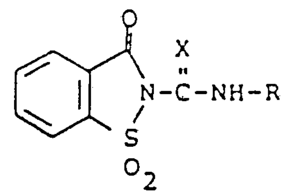

(I)

in welcher

X für Sauerstoff oder Schwefel steht,

R für die Gruppierung —CO—$R^1$ oder —$SO_2$—$OR^2$ steht, wobei

$R^1$ für geradkettiges oder verzweigtes Alkyl mit 1 bis 4 Kohlenstoffatomen, für geradkettiges oder verzweigtes Halogenalkyl oder Halogenalkoxy mit jeweils 1 bis 6 Kohlenstoffatomen und 1 bis 5 gleichen oder verschiedenen Halogenatomen, für geradkettiges oder verzweigtes Alkoxy oder Alkylthio mit jeweils 1 bis 10 Kohlenstoffatomen, für jeweils gegebenenfalls 1- bis 5-fach, gleich oder verschieden substituiertes Aryl, Aryloxy oder Arylthio mit jeweils 6 bis 10 Kohlenstoffatomen, wobei als Substituenten des Aryls genannt seien : Halogen, geradkettiges oder verzweigtes Alkoxy mit 1 bis 4 Kohlenstoffatomen, Alkoxycarbonyl mit 1 bis 4 Kohlenstoffatomen im Alkoxyteil, geradkettiges oder verzweigtes Alkyl mit 1 bis 4 Kohlenstoffatomen und N-Halogenalkyl-N-halogen-alkylthioamin mit 1 bis 3 Kohlenstoffatomen und 1 bis 5 gleichen oder verschiedenen Halogenatomen je Halogenalkylrest ; ferner für Cycloalkoxy mit 3 bis 6 Kohlenstoffatomen oder für die Gruppe —$NR^3R^4$ steht,

$R^2$ für geradkettiges oder verzweigtes Alkyl mit 1 bis 4 Kohlenstoffatomen oder Phenyl steht,

$R^3$ für geradkettiges oder verzweigtes Alkyl mit 1 bis 8 Kohlenstoffatomen steht und

$R^4$ für geradkettiges oder verzweigtes Alkyl mit 1 bis 4 Kohlenstoffatomen, Phenyl, Halogenalkylthio mit 1 bis 4 Kohlenstoffatomen und 1 bis 5 gleichen oder verschiedenen Halogenatomen, Alkoxycarbonyl mit 1 bis 4 Kohlenstoffatomen im Alkoxyteil oder Phenoxycarbonyl steht oder

$R^3$ und $R^4$ gemeinsam mit dem Stickstoffatom, an dem sie stehen, einen Saccharinrest bilden.

2. Acylierte Saccharin-Derivate der Formel (I) gemäß Anspruch 1, in welcher

X für Sauerstoff oder Schwefel steht,

R für die Gruppierung —CO—$R^1$ oder —$SO_2$—$OR^2$ steht, wobei

$R^1$ für Methyl, Ethyl, für geradkettiges oder verzweigtes Halogenalkyl oder Halogenalkoxy mit jeweils 1 bis 3 Kohlenstoffatomen und 1 bis 3 gleichen oder verschiedenen Fluor- und Chloratomen, für geradkettiges oder verzweigtes Alkoxy mit 1 bis 10 Kohlenstoffatomen, für geradkettiges oder verzweigtes Alkylthio mit 1 bis 4 Kohlenstoffatomen, für jeweils gegebenenfalls ein- bis dreifach, gleich oder verschieden durch Chlor, Fluor, Methoxy, Ethoxy, Methyl, Ethyl, Methoxycarbonyl, Ethoxycarbonyl und N-Trifluormethyl-N-fluordichlormethylthioamin substituiertes Phenyl, Phenoxy oder Phenylthio, für Cyclohexoxy oder für die Gruppe —$NR^3R^4$ steht,

$R^2$ für Methyl, Ethyl oder Phenyl steht,

$R^3$ für geradkettiges oder verzweigtes Alkyl mit 1 bis 5 Kohlenstoffatomen steht,

$R^4$ für Methyl, Ethyl, Phenyl, Phenoxycarbonyl, Methoxycarbonyl, Ethoxycarbonyl oder Halogenalkylthio mit 1 bis 3 Kohlenstoffatomen und 1 bis 3 gleichen oder verschiedenen Fluor- und Chloratomen steht oder

$R^3$ und $R^4$ mit dem Stickstoffatom, an dem sie stehen, einen Saccharinrest bilden.

3. Acylierte Saccharin-Derivate der Formel (I) gemäß Anspruch 1, in welcher

X für Sauerstoff steht,

R für die Gruppierung —CO—$R^1$ steht, wobei

$R^1$ für Methoxy, Ethoxy, n-Propoxy, iso-Propoxy, n-Butoxy, iso-Butoxy, tert.-Butoxy, Pentoxy, Hexoxy, 2-n-Butyl-butoxy, Chlormethoxy, 2,2,2-Trifluorethoxy, 1-Chlormethyl-2-chlorethoxy, Methylthio, Ethylthio, n-Propylthio, iso-Propylthio, Phenoxy, Phenylthio, Cyclohexoxy, 2-Methoxy-phenoxy, 4-Methoxy-phenoxy, 2-Chlor-phenoxy, 4-Chlorphenoxy, 2,4-Dichlorphenoxy, 2-Methoxycarbonyl-phenoxy, 2-Chlorphenylthio, 4-Chlorphenylthio, N-Methyl-N-phenylamino, N-Ethyl-N-phenylamino, N-n-Propyl-N-phenyl-amino, N-n-Butyl-N-phenyl-amino, N-n-Butyl-N-trichlormethylthio-amino, N-Methyl-N-phenoxy-carbonyl-amino, N-Ethyl-N-phenoxy-carbonyl-amino, N-n-Propyl-N-phenoxy-carbonyl-amino, N-Methyl-N-methoxy-carbonyl-amino, N-Ethyl-N-ethoxy-carbonyl-amino, N-n-Butyl-N-phenoxy-carbonyl-amino, N-iso-Butyl-N-phenoxy-carbonyl-amino, N-iso-Pentyl-N-phenoxy-carbonyl-amino, 2-[N-Trifluormethyl-N-dichlor-fluormethylthio-amino]-phenyl, 3-[N-Trifluormethyl-N-dichlorfluormethylthio-amino]-phenyl, 4-[N-Trifluormethyl-N-dichlorfluor-methylthio-amino]-phenyl oder 1,1-Dioxid-3-keto-2H, 3H-1,2-benzisothiazolyl steht, oder

X für Sauerstoff steht,

R für die Gruppierung $—SO_2—OR^2$ steht, wobei

$R^2$ für Phenyl, Methyl oder Ethyl steht.

4. Verfahren zur Herstellung von acylierten Saccharin-Derivaten der allgemeinen Formel (I)

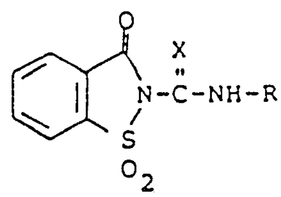

(I)

in welcher

X für Sauerstoff oder Schwefel steht,

R für die Gruppierung $—CO—R^1$ oder $—SO_2—OR^2$ steht, wobei

$R^1$ für geradkettiges oder verzweigtes Alkyl mit 1 bis 4 Kohlenstoffatomen, für geradkettiges oder verzweigtes Halogenalkyl oder Halogenalkoxy mit jeweils 1 bis 6 Kohlenstoffatomen und 1 bis 5 gleichen oder verschiedenen Halogenatomen, für geradkettiges oder verzweigtes Alkoxy oder Alkylthio mit jeweils 1 bis 10 Kohlenstoffatomen, für jeweils gegebenenfalls 1- bis 5-fach, gleich oder verschieden substituiertes Aryl, Aryloxy oder Arylthio mit jeweils 6 bis 10 Kohlenstoffatomen, wobei als Substituenten des Aryls genannt seien: Halogen, geradkettiges oder verzweigtes Alkoxy mit 1 bis 4 Kohlenstoffatomen, Alkoxycarbonyl mit 1 bis 4 Kohlenstoffatomen im Alkoxyteil, geradkettiges oder verzweigtes Alkyl mit 1 bis 4 Kohlenstoffatomen und N-Halogenalkyl-N-halogenalkylthioamin mit 1 bis 3 Kohlenstoffatomen und 1 bis 5 gleichen oder verschiedenen Halogenatomen je Halogenalkylrest: ferner für Cycloalkoxy mit 3 bis 6 Kohlenstoffatomen oder für die Gruppe $—NR^3R^4$ steht,

$R^2$ für geradkettiges oder verzweigtes Alkyl mit 1 bis 4 Kohlenstoffatomen oder Phenyl steht,

$R^3$ für geradkettiges oder verzweigtes Alkyl mit 1 bis 8 Kohlenstoffatomen steht und

$R^4$ für geradkettiges oder verzweigtes Alkyl mit 1 bis 4 Kohlenstoffatomen, Phenyl, Halogenalkylthio mit 1 bis 4 Kohlenstoffatomen und 1 bis 5 gleichen oder verschiedenen Halogenatomen, Alkoxycarbonyl mit 1 bis 4 Kohlenstoffatomen im Alkoxyteil oder Phenoxycarbonyl steht oder

$R^3$ und $R^4$ gemeinsam mit dem Stickstoffatom, an dem sie stehen, einen Saccharinrest bilden, dadurch gekennzeichnet, daß man Saccharin der Formel (II)

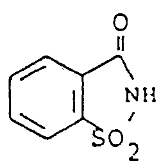

(II)

mit Iso- bzw. Thioisocyanat-Derivaten der Formel (III)

$$XCN—R \quad (III)$$

in welcher

X und R die oben angegebene Bedeutung haben, gegebenenfalls in Gegenwart eines Lösungs- oder Verdünnungsmittel und gegebenenfalls in Gegenwart eines Katalysators umsetzt.

5. Schädlingsbekämpfungsmittel, gekennzeichnet durch einen Gehalt an mindestens einem acylierten Saccharin-Derivat der Formel (I) gemäß den Ansprüchen 1 bis 3.

6. Verfahren zur Bekämpfung von Schädlingen, dadurch gekennzeichnet, daß man acylierte Saccharin-Derivate der Formel (I) gemäß den Ansprüchen 1 bis 3 auf Schädlinge und/oder ihren Lebensraum einwirken läßt.

7. Verwendung von acylierten Saccharin-Derivaten der Formel (I) gemäß den Ansprüchen 1 bis 3 zur Bekämpfung von Schädlingen.

8. Verfahren zur Herstellung von Schädlingsbekämpfungsmitteln, dadurch gekennzeichnet, daß man acylierte Saccharin-Derivate der Formel (I) gemäß den Ansprüchen 1 bis 3 mit Streckmitteln und/oder oberflächenaktiven Mitteln mischt.

9. Verwendung von acylierten Saccharin-Derivaten gemäß Ansprüchen 1 bis 3 zur Bekämpfung von Pilzen und Bakterien.

**Claims**

1. Acylated saccharin derivatives of the general formula (I)

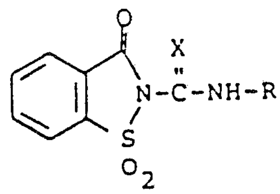

(I)

in which

X represents oxygen or sulphur and

R represents the grouping —CO—$R^1$ or —$SO_2$—$OR^2$,

wherein

$R^1$ represents straight-chain or branched alkyl with 1 to 4 carbon atoms, or represents straight-chain or branched halogenoalkyl or halogenoalkoxy with in each case 1 to 6 carbon atoms and 1 to 5 identical or different halogen atoms, or represents straight-chain or branched alkoxy or alkylthio with in each case 1 to 10 carbon atoms, or represents aryl, aryloxy or arylthio with in each case 6 to 10 carbon atoms, each of which is optionally mono-, di-, tri-, tetra- or penta-substituted by identical or different substituents, substituents of the aryl which may be mentioned being : halogen, straight-chain or branched alkoxy with 1 to 4 carbon atoms, akoxycarbonyl with 1 to 4 carbon atoms in the alkoxy part, straight-chain or branched alkyl with 1 to 4 carbon atoms and N-halogenoalkyl-N-halogenoalkylthioamine with 1 to 3 carbon atoms and 1 to 5 identical or different halogen atoms per halogenoalkyl radical ; or furthermore represents cycloalkoxy with 3 to 6 carbon atoms or represents the group —$NR^3R^4$.

$R^2$ represents straight-chain or branched alkyl with 1 to 4 carbon atoms or phenyl,

$R^3$ represents straight-chain or branched alkyl with 1 to 8 carbon atoms and

$R^4$ represents straight-chain or branched alkyl with 1 to 4 carbon atoms, phenyl, halogenoalkylthio with 1 to 4 carbon atoms and 1 to 5 identical or different halogen atoms, alkoxycarbonyl with 1 to 4 carbon atoms in the alkoxy part or phenoxycarbonyl, or

$R^3$ and $R^4$, together with the nitrogen atom on which they stand, form a saccharin radical. Acylated saccharin derivatives of the formula (I) according to Claim 1,

in which

X represents oxygen or sulphur and

R represents the grouping —CO—$R^1$ or —$SO_2$—$OR^2$,

wherein

$R^1$ represents methyl or ethyl, or represents straight-chain or branched halogenoalkyl or halogenoalkoxy with in each case 1 to 3 carbon atoms and 1 to 3 identical or different fluorine and chlorine atoms, or represents straight-chain or branched alkoxy with 1 to 10 carbon atoms, or represents straight-chain or branched alkylthio with 1 to 4 carbon atoms, or represents phenyl, phenoxy or phenylthio, each of which is optionally mono-, di- or tri-substituted by identical or different substituents from the group comprising chlorine, fluorine, methoxy, ethoxy, methyl, ethyl, methoxycarbonyl, ethoxycarbonyl and N-trifluoromethyl-N-fluorodichloromethylthioamino, or represents cyclohexoxy or represents the group —$NR^3R^4$,

$R^2$ represents methyl, ethyl or phenyl,

$R^3$ represents straight-chain or branched alkyl with 1 to 5 carbon atoms and

$R^4$ represents methyl, ethyl, phenyl, phenoxycarbonyl, methoxycarbonyl, ethoxycarbonyl or halogenoalkylthio with 1 to 3 carbon atoms and 1 to 3 identical or different fluorine and chlorine atoms, or

$R^3$ and $R^4$, with the nitrogen atom on which they stand, form a saccharin radical.

3. Acylated saccharin derivatives of the formula (I) according to Claim 1,

in which

X represents oxygen and

R represents the grouping —CO—$R^1$,

wherein

$R^1$ represents methoxy, ethoxy, n-propoxy, iso-propoxy, n-butoxy, iso-butoxy, tert.-butoxy, pentoxy, hexoxy, 2-n-butyl-butoxy, chloromethoxy, 2,2,2-trifluoroethoxy, 1-chloromethyl-2-chloro-ethoxy, methylthio, ethylthio, n-propylthio, iso-propylthio, phenoxy, phenylthio, cyclohexoxy, 2-methoxy-phenoxy, 4-methoxy-phenoxy, 2-chlorophenoxy, 4-chlorophenoxy, 2,4-dichlorophenoxy, 2-methoxycarbonyl-phenoxy, 2-chlorophenylthio, 4-chlorophenylthio, N-methyl-N-phenylamino, N-ethyl-N-phenylamino, N-n-propyl-N-phenyl-amino, N-n-butyl-N-phenyl-amino, N-n-butyl-N-trichloromethylthio-amino, N-methyl-N-phenoxycarbonyl-amino, N-ethyl-N-phenoxy-carbonyl-amino, N-n-propyl-N-phenoxy-carbonyl-amino, N-methyl-N-methoxy-carbonyl-amino, N-ethyl-N-ethoxy-carbonyl-amino, N-n-butyl-N-phenoxy-carbonyl-amino, N-iso-butyl-N-phenoxy-carbonyl-amino, N-iso-pentyl-N-phenoxy-carbonyl-amino, 2-[N-trifluoromethyl-N-dichloro-fluoromethylthio-amino]-phenyl, 3-[N-trifluoromethyl-N-dichlorofluoromethylthio-amino]-phenyl, 4-[N-trifluoromethyl-N-dichlorofluoromethylthio-amino]-phenyl or 1,1-dioxide-3-keto-2H,3H-1,2-benzisothiazolyl, or

X represents oxygen and

R represents the grouping $—SO_2—OR^2$,

wherein

$R^2$ represents phenyl, methyl or ethyl.

4. Process for the preparation of acylated saccharin derivatives of the general formula (I)

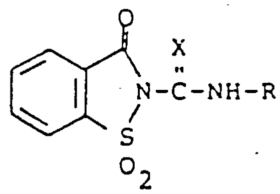

(I)

in which

X represents oxygen or sulphur and

R represents the grouping $—CO—R^1$ or $—SO_2—OR^2$,

wherein

$R^1$ represents straight-chain or branched alkyl with 1 to 4 carbon atoms, or represents straight-chain or branched halogenoalkyl or halogenoalkoxy with in each case 1 to 6 carbon atoms and 1 to 5 identical or different halogen atoms, or represents straight-chain or branched alkoxy or alkyl thio with in each case 1 to 10 carbon atoms, or represents aryl, aryloxy or arylthio with in each case 6 to 10 carbon atoms, each of which is optionally mono-, di-, tri-, tetra- or penta-substituted by identical or different substituents, substituents of the aryl which may be mentioned being : halogen, straight-chain or branched alkoxy with 1 to 4 carbon atoms, alkoxycarbonyl with 1 to 4 carbon atoms in the alkoxy part, straight-chain or branched alkyl with 1 to 4 carbon atoms and N-halogenoalkyl-N-halogenoalkylthioamine with 1 to 3 carbon atoms and 1 to 5 identical or different halogen atoms per halogenoalkyl radical ; or furthermore represents cycloalkoxy with 3 to 6 carbon atoms or represents the group $—NR^3R^4$,

$R^2$ represents straight-chain or branched alkyl with 1 to 4 carbon atoms or phenyl,

$R^3$ represents straight-chain or branched alkyl with 1 to 8 carbon atoms and

$R^4$ represents straight-chain or branched alkyl with 1 to 4 carbon atoms, phenyl, halogenoalkylthio with 1 to 4 carbon atoms and 1 to 5 identical or different halogen atoms, alkoxycarbonyl with 1 to 4 carbon atoms in the alkoxy part or phenoxycarbonyl, or

$R^3$ and $R^4$, together with the nitrogen atom on which they stand, form a saccharin radical, characterised in that saccharin of the formula (II)

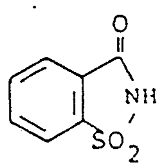

(II)

is reacted with iso- or thioiso-cyanate derivatives of the formula (III)

XCN—R (III)

in which

X and R have the abovementioned meaning, if appropriate in the presence of a solvent or diluent and if appropriate in the presence of a catalyst.

5. Agents for combating pests, characterised in that they contain at least one acylated saccharin derivative of the formula (I) according to Claims 1 to 3.

6. Method of combating pests, characterised in that acylated saccharin derivatives of the formula (I) according to Claims 1 to 3 are allowed to act on pests and/or their environment.

7. Use of acylated saccharin derivatives of the formula (I) according to Claims 1 to 3 combating pests.

8. Process for the preparation of agents for combating pests, characterised in that acylated saccharin derivatives of the formula (I) according to Claims 1 to 3 are mixed with extenders and/or surface-active agents.

9. Use of acylated saccharin derivatives according to Claims 1 to 3 for combating fungi and bacteria.

**Revendications**

1. Dérivés acylés de la saccharine de formule générale

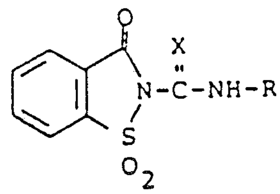

(I)

dans laquelle

X représente l'oxygène ou le soufre,

R représente le groupement $—CO—R^1$ ou $—SO_2—OR^2$ dans lequel

$R^1$ représente un groupe alkyle à chaîne droite ou ramifiée en $C_1$-$C_4$, un groupe halogénoalkyle ou halogénoalcoxy à chaîne droite ou ramifiée contenant chacun 1 à 6 atomes de carbone et 1 à 5 atomes d'halogènes identiques ou différents, un groupe alcoxy ou alkylthio à chaîne droite ou ramifiée contenant chacun 1 à 10 atomes de carbone, un groupe aryle, aryloxy ou arylthio en $C_6$-$C_{10}$ portant éventuellement 1 à 5 substituants identiques ou différents, les substituants du groupe aryle étant des halogènes, des groupes alcoxy à chaîne droite ou ramifiée en $C_1$-$C_4$, alcoxycarbonyle contenant 1 à 4 atomes de carbone dans la partie alcoxy, alkyle à chaîne droite ou ramifiée en $C_1$-$C_4$ et N-halogénoalkyl-N-halogénoalkylthioamino contenant 1 à 3 atomes de carbone et 1 à 5 atomes d'halogènes identiques ou différents par groupe halogénoalkyle ; ou encore un groupe cycloalcoxy en $C_3$-$C_6$ ou le groupe $—NR^3R^4$,

$R^2$ représente un groupe alkyle à chaîne droite ou ramifiée en $C_1$-$C_4$ ou un groupe phényle,

$R^3$ représente un groupe alkyle à chaîne droite ou ramifiée en $C_1$-$C_8$, et

$R^4$ représente un groupe alkyle à chaîne droite ou ramifiée en $C_1$-$C_4$- phényle, halogénoalkylthio contenant 1 à 4 atomes de carbone et 1 à 5 atomes d'halogènes identiques ou différents, alcoxycarbonyle contenant 1 à 4 atomes de carbone dans la partie alcoxy ou phénoxycarbonyle, ou bien

$R^3$ et $R^4$ forment ensemble et avec l'atome d'azote auquel ils sont reliés un radical de saccharine.

2. Dérivés acylés de la saccharine de formule I selon la revendication 1, dans laquelle

X représente l'oxygène ou le soufre,

R représente un groupement $—CO—R^1$ ou $—SO_2—OR^2$ dans lequel

$R^1$ représente un groupe méthyle, éthyle, halogénoalkyle ou halogénoalcoxy à chaîne droite ou ramifiée contenant chacun 1 à 3 atomes de carbone et 1 à 3 atomes identiques ou différents de fluor ou de chlore, un groupe alcoxy à chaîne droite ou ramifiée en $C_1$-$C_{10}$, un groupe alkylthio à chaîne droite ou ramifiée en $C_1$-$C_4$, un groupe phényle, phénoxy ou phénylthio portant éventuellement 1 à 3 substituants identiques ou différents choisis parmi le chlore, le fluor, les groupes méthoxy, éthoxy, méthyle, éthyle, méthoxy-carbonyle, éthoxycarbonyle, et N-trifluorométhyl-N-fluorodichlorométhylthioamino, un groupe cyclohexoxy ou le groupe $—NR^3R^4$,

$R^2$ représente un groupe méthyle, éthyle ou phényle,

$R^3$ représente un groupe alkyle à chaîne droite ou ramifiée en $C_1$-$C_5$,

$R^4$ représente un groupe méthyle, éthyle, phényle, phénoxycarbonyle, méthoxycarbonyle, éthoxycarbonyle ou halogénoalkylthio contenant 1 à 3 atomes de carbone et 1 à 3 atomes identiques ou différents de fluor ou de chlore, ou bien

$R^3$ et $R^4$ forment avec l'atome d'azote auquel ils sont reliés un radical de saccharine.

3. Dérivés acylés de la saccharine de formule I selon la revendication 1, dans laquelle

X représente l'oxygène,

R représente le groupement $—CO—R^1$ dans lequel

$R^1$ représente un groupe méthoxy, éthoxy, n-propoxy, isopropoxy, n-butoxy, isobutoxy, tert-butoxy, pentoxy, hexoxy, 2-n-butyl-butoxy, chlorométhoxy, 2,2,2-trifluoréthoxy, 1-chlorométhyl-2-chloréthoxy, méthylthio, éthylthio, n-propylthio, isopropylthio, phénoxy, phénylthio, cyclohexoxy, 2-méthoxy-phénoxy, 4-méthoxy-phénoxy, 2-chlorophénoxy, 4-chlorophénoxy, 2,4-dichlorophénoxy, 2-méthoxycarbonyl-phénoxy, 2-chlorophénylthio, 4-chlorophénylthio, N-méthyl-N-phénylamino, N-éthyl-N-phénylamino, N-n-propyl-N-phénylamino, N-n-butyl-N-phénylamino, N-n-butyl-N-trichlorométhylthio-amino, N-méthyl-N-phénoxycarbonylamino, N-éthyl-N-phénoxycarbonylamino, N-n-propyl-N-phénoxycarbonylamino, N-méthyl-N-méthoxycarbonylamino, N-éthyl-N-éthoxycarbonylamino, N-n-butyl-N-phénoxycarbonylamino, N-isobutyl-N-phénoxycarbonylamino, N-isopentyl-N-phénoxycarbonylamino, 2-(N-trifluorométhyl-N-dichlorofluorométhylthio-amino)-phényle, 3-(N-trifluorométhyl-N-dichlorofluorométhylthio-amino)-phényle, 4-(N-trifluorométhyl-N-dichlorofluorométhylthio-amino)-phényle ou 1,1-dioxyde de 3-céto-2H,3H-1,2-benzoisothiazolyle, ou bien

X représente l'oxygène,

R représente le groupe $—SO_2—OR^2$ dans lequel

$R^2$ représente un groupe phényle, méthyle ou éthyle.

4. Procédé de préparation de dérivés acylés de la saccharine répondant à la formule générale I

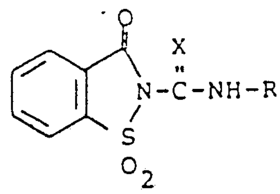

(I)

dans laquelle

X représente l'oxygène ou le soufre,

R représente un groupement —CO—$R^1$ ou —$SO_2OR^2$ dans lequel

$R^1$ représente un groupe alkyle à chaîne droite ou ramifiée en $C_1$-$C_4$, un groupe halogénoalkyle ou halogénoalcoxy à chaîne droite ou ramifiée contenant chacun 1 à 6 atomes de carbone et 1 à 5 atomes d'halogènes identiques ou différents, un groupe alcoxy ou alkylthio à chaîne droite ou ramifiée contenant chacun 1 à 10 atomes de carbone, un groupe aryle, aryloxy ou arylthio contenant chacun 6 à 10 atomes de carbone et éventuellement 1 à 5 substituants identiques ou différents, les substituants du groupe aryle étant des halogènes, des groupes alcoxy à chaîne droite ou ramifiée en $C_1$-$C_4$, alcoxycarbonyle contenant 1 à 4 atomes de carbone dans la partie alcoxy, alkyle à chaîne droite ou ramifiée en $C_1$-$C_4$ et N-halogénoalkyl-N-halogénoalkylthiamino contenant 1 à 3 atomes de carbone et 1 à 5 atomes d'halogènes identiques ou différents par groupe halogénoalkyle ; ou encore un groupe cycloalcoxy en $C_3$-$C_6$ ou le groupe —$NR^3R^4$,

$R^2$ représente un groupe alkyle à chaîne droite ou ramifiée en $C_1$-$C_4$ ou phényle,

$R^3$ représente un groupe alkyle à chaîne droite ou ramifiée en $C_1$-$C_8$, et

$R^4$ représente un groupe alkyle à chaîne droite ou ramifiée en $C_1$-$C_4$, phényle, halogénoalkylthio contenant 1 à 4 atomes de carbone et 1 à 5 atomes d'halogènes identiques ou différents, alcoxycarbonyle contenant 1 à 4 atomes de carbone dans la partie alcoxy ou phénoxycarbonyle, ou bien

$R^3$ et $R^4$ forment ensemble et avec l'atome d'azote auquel ils sont reliés un radical de saccharine, caractérisé en ce que l'on fait réagir la saccharine de formule II

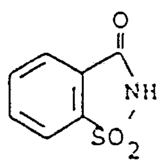

(II)

avec des iso- ou thioisocyanates de formule III

XCN—R (III)

dans laquelle X et R ont les significations indiquées ci-dessus, éventuellement en présence d'un solvant ou diluant et éventuellement en présence d'un catalyseur.

5. Produit pesticide, caractérisé en ce qu'il contient au moins un dérivé acylé de la saccharine de formule I selon les revendications 1 à 3.

6. Procédé pour combattre les parasites, caractérisé en ce que l'on fait agir des dérivés acylés de la saccharine de formule I selon les revendications 1 à 3 sur les parasites et/ou leur habitat.

7. Utilisation des dérivés acylés de la saccharine de formule I selon les revendications 1 à 3 dans la lutte contre les parasites.

8. Procédé de préparation de produits pesticides, caractérisé en ce que l'on mélange des dérivés acylés de la saccharine de formule I selon les revendications 1 à 3 avec les diluants et/ou des agents tensioactifs.

9. Utilisation de dérivés acylés de la saccharine selon les revendications 1 à 3 dans la lutte contre les mycètes et les bactéries.